# EUROPÄISCHE PATENTSCHRIFT

(11) **EP 1 322 559 B1**
(45) Veröffentlichungstag und Bekanntmachung des Hinweises auf die Patenterteilung: **03.01.2007**
(21) Anmeldenummer: 01969772.1
(22) Anmeldetag: 25.09.2001
(51) Int. Cl.: C02F 3/10, C02F 3/34, C12N 11/04

(54) **VERWENDUNG EINER MIKROBIOLOGISCHEN KULTUR FÜR DIE EINLEITUNG VON MIKROBIOLOGISCHEN ABLÄUFEN IN DER AQUARISTIK**
USE OF A MICROBIOLOGICAL CULTURE FOR TRIGGERING MICROBIOLOGICAL PROCESSES IN AQUARISTICS
UTILISATION D'UNE CULTURE MICROBIOLOGIQUE SERVANT A AMORCER DES PROCESSUS MICROBIOLOGIQUES DANS DES AQUARIUMS

(30) Priorität: 25.09.2000 DE 10047709
(43) Veröffentlichungstag der Anmeldung: 02.07.2003
(73) Patentinhaber: Söll Holding GmbH, 61476 Kronberg (DE)
(72) Erfinder: WILLUWEIT, Thomas, 95028 Hof (DE); SÖLL, Peter, 95028 Hof (DE); MÜLLER, Robert, 95028 Hof (DE)
(74) Vertreter: Christophersen, Ruth
(86) Internationale Anmeldenummer: PCT/EP2001/011090
(87) Internationale Veröffentlichungsnummer: WO 2002/024583

(56) Entgegenhaltungen:
- DE-A- 3 928 255
- DE-A- 4 027 221
- DE-A- 4 028 312
- US-A- 6 153 416

## Beschreibung

Die vorliegende Erfindung betrifft die Verwendung einer mikrobiologischen Kultur für die Einleitung von mikrobiologischen Abläufen in der Aquaristik enthaltend lebende chemolithoautotrophen Bakterien, die immobilisiert sind.

Stehende und fließende natürliche Gewässer weisen in der Regel eine gewisse Selbstreinigungskraft auf, d.h. Verunreinigungen können in beschränktem Umfang abgebaut werden. Diese Selbstreingungskräfte reichen bei stark belasteten Gewässern oft nicht aus. Diesen Gewässern werden zur Unterstützung der (Selbst)Reinigung häufig Mikroorganismen zugesetzt, die insbesondere schädliche Stickstoffverbindungen in unbedenkliche Verbindungen, wie elementaren Stickstoff abbauen können.

Die zugesetzten Mikroorganismen dienen dazu, die in natürlichen Gewässern und aquatischen Systemen vorhandenen Selbstreinigungskräfte anzustoßen und zu unterstützen bzw. zu übernehmen.

Die Mikroorganismen können in Form von Zellsuspehsionen eingesetzt und in dieser Form, gegebenenfalls mit einem wässerigen Lösungsmittel, in die Gewässer eingebracht werden. Auch der Einsatz von pulverförmigen Mikroorganismen ist möglich. In diesen Zubereitungen werden die Mikroorganismen zunächst in sogenannte Dauerformen, wie Sporen überführt oder auch lyophilisiert. Die Mikroorganismen bleiben aufgrund der gewässereigenen Strömung und der Diffusion nicht an Ort und Stelle sondern gelangen auch in andere Regionen des Gewässers. In diesen Regionen können die Lebensbedingungen für die Mikroorganismen ungünstig sein, so dass eine Schadstoffentfernung nicht stattfinden kann. Ferner ist eine Fixierung der Mikroorganismen in besonders belasteten Gewässerregionen nicht möglich.

Im Bereich der Aquaristik treten schon bei geringen Mengen an löslichen Stickstoffverbindungen Probleme auf, da z. B. Ammoniak bereits ab einer Konzentration von 0,01 mg/L toxisch auf Fische wirkt. Insbesondere in Aquarien, die künstliche Systeme darstellen, ist der Selbstreinigungsprozeß bereits bei geringen Mengen an enthaltenen Fremdsubstanzen, wie Stickstoffverbindungen etc., besonders störanfällig.

Insbesondere wenn in den aufzubereitenden Gewässern kaum aktive Mikroorganismen vorliegen müssen aktive Mikroorganismen in ausreichend hoher Aktivkonzentration zugesetzt werden, um die Selbstreinigungskräfte der Gewässer zu aktivieren. Für den Betreiber von Gartenteichen und kleineren Gewässern sind Mikroorganismen, die zur Verbesserung der Wasserqualität eingesetzt werden, in der Regel in sogenannten Dauerformen erhältlich oder lyophilisiert. Für größere Gewässer bietet sich der Einsatz von Zellsuspensionen an. Es ist auch bekannt, die Mikroorganismen in verkapselter Form einzusetzen.

Die aus dem Stand der Technik bekannten Zellsuspensionen und auch die Mikrokapseln, welche Mikroorganismen enthalten, setzen in der Regel keine chemoautotrophen Mikroorganismen in definierten Artengemeinschaften ein. Auch haben sie den Nachteil, dass die Zubereitungen nur über einen sehr kurzen Zeitraum lagerbeständig sind und die Mikroorganismen bereits nach ein bis zwei Tagen absterben. Derartige Zubereitungen sind also für den Handel nicht geeignet.

Sollen Mikroorganismen zur Impfung von Gewässern eingesetzt werden, um deren Selbstreinigungskräfte zu aktivieren, müssen die eingesetzten Mikroorganismen in sehr hoher Konzentration vorliegen.

Bei der Stabilisierung des Stickstoffkreislaufes bereiten mikrobiologische Prozesse wie die Stickstofffixierung, Stickstoffassimilation und die Denitrifikation in der Regel keine Probleme, die Abbauleistungen aus Nitrifikationsprozessen und auch die Mineralisierung organischer Stickstoffverbindungen sind häufig nicht ausreichend.

Bei vielen mikrobiologischen Prozessen ist nachteilig, dass der Aktivitätszeitraum der Mikroorganismen nur wenige Tage beträgt und diese dann absterben. Hinzukommt, dass viele Mikroorganismen keine Sporen bilden und nicht in Dauerformen überführt werden können, beispielsweise können sie nur unter Verlust der Aktivität lyophilisiert werden. Diese Mikroorganismen müssen in vegetativer Form eingesetzt werden, was wiederum bedeutet, dass sie unter Erhaltungsbedingungen gelagert werden müssen.

Der vorliegenden Erfindung lag die Aufgabe zugrunde, mikrobiologischen Starterkulturen zur Verfügung zu stellen, die über einen gewissen Zeitraum gelagert werden können und dazu geeignet sind, in der Aquaristik mikrobiologische Abläufe zu starten und somit die Selbstreinigungskraft dieses Systems anzustoßen.

Gegenstand der vorliegenden Erfindung ist demgemäß die Verwendung einer mikrobiologischen Kultur für die Einleitung von mikrobiologischen Abläufen in der Aquaristik enthaltend lebende chemolithoautotrophe Bakterien, die immobilisiert sind, **dadurch gekennzeichnet, dass** die Bakterien in Reinkultur angezüchtet werden und ein Bakteriengemisch aus a) ammoniakoxidierenden und b) nitritoxidierenden Bakterien eingesetzt wird, wobei das Artenverhältnis der Zellzahlen im Immobilisat von a:b in dem Bereich von 1:10.000 bis 1:1 liegt.

Immobilisiert bedeutet im Rahmen der vorliegenden Erfindung, daß die Bakterien, im folgenden auch Mikroorganismen genannt, nicht in Form einer Zellsuspension bzw. -lösung als solche eingesetzt werden, sondern daß sie von einem Matrixmaterial umgeben und/oder darauf aufgebracht sind, wobei die Erhaltungsbedingungen eingehalten werden. Die immobilisierten Bakterien werden im folgenden auch als lmmobilisate bezeichnet.

Überraschenderweise wurde festgestellt, dass die erfindungsgemäß verwendeten Bakterienkulturen in immobilisierter Form sich gut für das Starten von mikrobiologischen Prozessen in der Aquaristik eignen und so die Reinigungsprozesse in diesen Systemen deutlich beschleunigt werden können. Wasser aus dem zu reinigenden System kommt mit den Mikroorganismen in Kontakt und es findet somit auch ein Kontakt zwischen den schädlichen Substanzen und den Mikroorganismen unter Abbau dieser Substanzen statt. Die erfindungsgemäße Starterkultur eignet sich insbesondere zum Abbau von organischen und anorganischen Stickstoffverbindungen.

Ein weiterer Vorteil der Immobilisation der Mikroorganismen liegt darin, dass diese während der Lagerung mit frischem Medium, mit Nährstoffen und Sauerstoff versorgt werden können und somit vor einem Verlust an Aktivität geschützt werden. Ferner ist es möglich, die Mikroorganismen auch über einen längeren Zeitraum zu lagern, so daß sie nicht, wie die aus dem Stand der Technik bekannten Systeme, unmittelbar nach ihrer Herstellung eingesetzt werden müssen.

Zum Bereich der Aquaristik, in dem die erfindungsgemäß verwendeten Bakterienkulturen eingesetzt werden können, zählen künstliche stehende und fließende Gewässer, wie Teiche und Aquarien.

Die erfindungsgemäßen Bakterienkulturen eignen sich insbesondere zur Verbesserung der Reinigungsleistung von Aquarien und Teichen.

Die Mikroorganismen sind vorzugsweise in einer Matrix immobilisiert, wobei sich als Matrixform Kapseln, Gele und/oder Gelkugeln besonders geeignet erwiesen haben. Diese Art der lmmobilisierung hat den Vorteil, daß Feuchtigkeit oder Wasser aus dem zu reinigenden System über eine in den Kapsel- bzw. Gelmaterialien vorliegende Netzstruktur oder Diffusion in das Immobilisat eindringen kann und so mit den Mikroorganismen in Kontakt kommt. Über die vorzugsweise für Wasser und/oder Mikroorganismen durchlässige Kapselwand bzw. das Gel werden gleichzeitig Mikroorganismen auch an die Umgebung abgegeben, so dass außerhalb der Kapseln ein Kontakt zwischen den schädlichen Substanzen und den Mikroorganismen unter Abbau dieser Substanzen stattfinden kann.

Die bevorzugt eingesetzten Kapseln bzw. Kugeln weisen vorzugsweise einen Durchmesser von etwa 100 bis etwa 10.000 µm, insbesondere von 100 bis 5.000 µm auf, worin die Mikroorganismen in fester oder flüssiger Form von einem festen bis gelartigen, in der Regel polymeren, vorzugsweise porösen Polymermaterial umhüllt, durchsetzt und/oder darauf aufgebracht sind.

Durch die Auswahl der Materialien für die Kapseln/Kugeln, wie natürlichen oder synthetischen Polymeren, kann die Kapsel/Kugel so gestaltet sein, daß die Bakterien und das zu reinigenden Substrat (Wasser) in Kontakt kommen können. Eine ggf. vorhandene Kapselwandung kann dicht, permeabel oder semipermeabel gestaltet werden. Das Material der Kapseln/Kugeln kann auch mehrschichtig aufgebaut sein, d.h. es können ein oder mehrere Materialien eingesetzt werden. Somit ergibt sich eine Fülle von Möglichkeiten, die immobilisierte Substanz gesteuert freizusetzen, z.B. durch Zerstören der Hülle oder durch Permeation oder auch durch chemische Reaktionen, die im Inneren der Mikrokapseln ablaufen können.

In einer bevorzugten Ausführungsform der vorliegenden Erfindung ist die Kapselwand für Wasser und die darin gelösten Substanzen durchlässig. In dieser Ausführungsform erfolgt der Kontakt der schädlichen Substanzen, mit den Mikroorganismen derart, dass das Wasser durch die Oberfläche in die Kugeln eindringt und im Inneren die der Reinigungsprozesse ablaufen. Durch den Reinigungsprozess vermehren sich die Mikroorganismen bis die Kapazitätsgrenze der Kapsel/Kugel bzw. des Gels erreicht ist und die Wandung aufbricht, d.h. die Mikroorganismen freigesetzt werden.

Zur Herstellung der immobilisierten Mikroorganismen können als Matrixmaterialien natürliche oder synthetische Polymere eingesetzt werden.

In einer bevorzugten Ausführungsform werden gelbildende Polymere und/oder solche Polymere, die zur Herstellung der bevorzugten Formen wie Kapseln, Kugeln und/oder Gele geeignet sind, eingesetzt. Diese haben den Vorteil, dass Bakterien innerhalb der Gelstruktur aufgenommen bzw. eingelagert werden können. Vorzugsweise sollten die Materialien jedoch eine solche Festigkeit und Abriebstabilität aufweisen, dass die immobilisierten Mikroorganismen in dieser Form unter sogenannten Erhaltungsbedingungen, d.h. unter Zusatz von Substrat und Sauerstoff, gelagert werden können.

In einer bevorzugten Ausführungsform werden solche Materialien eingesetzt, die sich in Wasser langsam, vorzugsweise in einer definierten Geschwindigkeit, auflösen beziehungsweise abgebaut werden, so dass langsam eine Freisetzung der Mikroorganismen über einen definierten Zeitraum erfolgt.

Weiterhin bevorzugt sind Polymere, die gegebenenfalls auch als Kohlenstoffquelle für die verwendeten Mikroorganismen dienen können. In dieser Ausführungsform wird die Matrix durch die Mikroorganismen abgebaut und zersetzt. Sobald die Struktur ausreichend große Poren aufweist, erfolgt eine Freisetzung der Mikroorganismen.

Beispiele für geeignete Polymere sind polymere Polysaccharide, wie Agarose oder Cellulose, Proteine, wie Gelatine, Gummi arabicum, Albumin oder Fibrinogen, Ethylcellulose, Methylcellulose, Carboxymethylethylcellulose, Celluloseacetate, Alkali-Cellulosesulfat Polyanillin, Polypyrrol, Polyvinylpyrolidon, Polystyrol, Polyvinylchlorid, Polyvinylalkohol, Polyethylen, Polypropylen, Copolymere aus Polystyrol und Maleinsäureanhydrid, Epoxidharze, Polyethylenimine, Copolymere aus Styrol und Methylmethacrylat, Polystyrolsulfonat, Polyacrylate und Polymethacrylate, Polycarbonate, Polyester, Silikone, Methylcellulose, Gemische aus Gelatine und Wasserglas, Gelatine und Polyphosphat, Celluloseacetat und Phthalat, Gelatine und Copolymeren aus Maleinsäureanhydrid und Methylvinylether, Celluloseacetatbutyrat, Chitosan, Polydialkyldimethylammoniumchlorid, Mischungen aus Polyacrylsäuren und Polydiallyldimethylammoniumchlorid sowie beliebige Gemische der voranstehenden.

Das Polymermaterial kann gegebenenfalls vernetzt sein. Übliche Vernetzer sind Glutaraldehyd, Harnstoff/Formaldehydharze, Taninverbindungen, wie Taninsäure, Erdalkaliionen, wie Ca²⁺-Ionen, die z. B. als Chloride zugesetzt werden können, und deren Gemische.

In einer besonders bevorzugten Ausführungsform werden Alginate und Alginatderivate als Matrixmaterial eingesetzt. Die Alginate haben den Vorteil, dass sie keinen negativen Einfluss auf die Aktivität der Mikroorganismen aufweisen, ferner können sie von Mikroorganismen über einen bestimmten Zeitraum, wie von einer Woche bis zu mehreren Monaten langsam abgebaut werden. Durch den langsamen Abbau der Matrix werden nach und nach die eingeschlossenen Mikroorganismen in höherem Maße freigesetzt. Aus der biologischen Abbaubarkeit des Wandmaterials ergibt sich der weitere Vorteil, dass im Gewässer keine Reststoffe beziehungsweise Abfallprodukte verbleiben. Werden die immobilisierten Bakterienkulturen z. B. in einem entsprechenden Behälter oder einer Vorrichtung zu ihrer Aufnahme und Lagerung in das zu behandelnde Medium gegeben, z. B. in einen Filter, kann dieser nach Auflösung der eingesetzten immobilisierten Mikroorganismen erneut mit den erfindungsgemäßen Mikroorganismen gefüllt werden.

Liegt das Matrixmaterial in Form einer Kapsel oder Kugel vor, so weist die Wandung in einer weiteren bevorzugten Ausführungsform einen mehrschichtigen Aufbau auf, wobei gelbildende Polymere und nicht-gelbildende, vorzugsweise filmbildende Polymere für die äußere Wandung kombiniert werden können. In einer bevorzugten Ausführungsform werden als ein gelbildende Materialien Alginate oder Alginatderivate eingesetzt und als weitere, vorzugsweise filmbildende Materialien Polymere ausgewählt aus Cellulosederivaten, insbesondere Natrium-Cellulosesulfat, Polydialkyldimethylammoniumchloriden und/oder Polyethyleniminen. Die Polymermaterialien, die neben dem Alginat beziehungsweise den Alginatderivaten eingesetzt werden, bilden vorzugsweise die äußere Kapselwand. Es wurde festgestellt, dass durch die Verwendung eines äußeren Kapselmaterials ausgewählt aus den voranstehend genannten Polymeren die Abriebstabilität der Mikrokapseln und somit die Lagerfähigkeit deutlich verbessert werden kann.

In einer besonders bevorzugten Ausführungsform werden als gelbildende Materialien aufgereinigte Alginate, insbesondere die unter den CAS-Nummern 9005-38-3 und 9005-32-7 beschriebenen Alginate verwendet. Die aufgereinigten Alginate haben den Vorteil, dass sie nur geringe Mengen an freien organischen Substanzen enthalten, welche ggf. die Stabilität und Aktivität der Mikroorganismen beeinträchtigen können. Die eingesetzten Alginate haben bevorzugt einen hohen Anteil an L-Guluronsäure-Einheiten.

In einer weiteren, bevorzugten Ausführungsform werden dem Alginat Schicht- und Gerüstsilikate, besonders bevorzugt Zeolithe zugesetzt. Mit ihren Gitterstrukturen stabilisieren die Silikate das Gelmaterial und verlangsamen den Zersetzungsprozess des Alginats bei gleichzeitiger Adsorption von Ammonium und Calcium an die mineralische Komponente der Kugelmatrix.

Die eingesetzten Zeolithe bestehen zu über 70 % aus Klinoptilotith mit inerten Beimineralien wie Quarz. Die Korngröße der mineralischen Beimengungen ist kleiner 600 µm. Die Beimengung beträgt 0,5 bis 50 Massenprozent der eingesetzten Alginattrockenmasse. Bevorzugt werden 5-30% w Mineralien, besonders bevorzugt w (Zeolith) =15-30 % zu 70 - 85 % Alginat eingesetzt.

Die Mikroorganismen sind vorzugsweise ausgewählt aus den Gattungen *Nitrosomonas,* Nitrosococcus, *Nitrosospira, Nitrosovibrio und Nitrosospira,* insbesondere die Arten Nitrosomonas halophila, *Nitrosomonas eutropha* und *Nitrosomonas europaea,* Nitrosomonas oligotropha, Nitrosomonas ureae, Nitrosomonas aestuarii, Nitrosomonas marina, Nitrosomonas sp. 3 Nm 51, Nitrosomonas communis, Nitrosomonas nitrosa, Nitrosomonas sp. 1 Nm 33, Nitrosomonas sp. 2 Nm 41, Nitrosomonas cryotolerans, sowie die nitritoxidierenden Bakterien der Gattungen *Nitrobacter* und *Nitrospira,* insbesondere *Nitrobacter winogradskyi.*

Geeignet sind auch heterotrophe Denitrifikanten, wie *Paracocus* sp., insbesondere Paraccocus pantothrophas, und *Pseudomonas* sp. Es können auch beliebige Kombinationen, d. h. Mischkulturen, von Mikroorganismen eingesetzt werden. Durch den Einsatz von Mischkulturen können hinsichtlich der Aktivität und Abbauleistung synergistische Effekte erhalten werden. Beispiele für Mischkulturen sind z. B. Kombinationen der Arten Nitrosomonas und Nitrobacter sowie ggf. heterotrophe Mikroorganismen.

In einer besonders bevorzugten Ausführungsform werden Artengermeinschaften von verschiedenen Bakterien eingesetzt. Die eingesetzten Arten werden zunächst entsprechend ihren speziellen Anzuchtbedingungen in Reinkultur angezogen und anschließend immobilisiert werden. Die Anzucht der Bakterien in Reinkultur ermöglicht es, nahezu beliebige Artengemeinschaften in nahezu beliebigen Artenverhältnissen zusammenzustellen. Ein Beispiel für eine besonders bevorzugte Artengemeinschaft im lmmobilisat besteht aus a) ammoniakoxidierenden (z.B. Nitrosomonas) und b) nitritoxidierenden (z.B. Nitrobacter) und c) Nitrat- und nitritreduzierenden Bakterien (z.B. Paracoccus). Das Artenverhältnis der Zellzahlen im Immobilisat liegt im Bereich von a : b 1:10.000 zu 1:1 und bevorzugt von 1:1000 zu 1:10 und das Artenverhältnis von b : c bevorzugt zwischen 1000:1 bis 1:1 und besonders bevorzugt zwischen 100:1 bis 5:1 liegt.

Je nach Einsatzzweck kann der Fachmann aufgrund seines Fachwissens und ggf. nach Durchführung von Tests oder unter Einsatz von Computersimulationen die entsprechenden Arten und deren Verhältnis zueinander bestimmen.

Um ein System mikrobiologisch in kurzer Zeit starten zu können, hat es sich als sinnvoll erwiesen, wenn die Starterkulturen in ausreichend hoher Konzentration zugesetzt werden.

Zur Herstellung der erfindungsgemäß verwendeten immobilisierten Bakterien werden üblicherweise zunächst Zellsuspensionen in einer Konzentration von 1 x 10⁶ bis 5 x 10⁹ Zellen/ml in Reinkultur angezüchtet. Um Mikroorganismen in einer möglichst hohen Konzentration in der Mikrokapsel zu erhalten werden die erhaltenen Zellsuspensionen anschließend vorzugsweise auf 5 x 10⁸ bis 6 x 10⁹ Zellen/ml aufkonzentriert. Das Aufkonzentrieren kann nach üblichen aus dem Stand der Technik bekannten Filtrationsverfahren erfolgen.

Insbesondere wenn nitrifizierende Mikroorganismen immobilisiert werden, hat es sich als besonders geeignet erwiesen, die Mikroorganismen in Form von wässerigen Zellsuspensionen einzusetzen. In einer besonderes bevorzugten Ausführungsform werden stabilisierte Mikroorganismen eingesetzt, insbesondere solche der Anzucht und Stabilisierung gemäß dem in der deutschen Patentanmeldung 199 08 109.3-41 beschriebenen Verfahren durch Zusatz von NO und/oder NO₂ erfolgt.

Eine besonders gute Stabilisierung der Mikroorganismen kann erreicht werden, wenn diese als Zellsuspension eingesetzt werden, welche ein Puffersystem enthält. Beispiele für geeignete Puffer sind Essigsäure/Acetat, HCO₃⁻/CO₃²⁻, Phosphorsäure/H₂PO₃⁻/HPO₃²⁻, Citronensäure/Citrat, Milchsäure/Lactat, festes CaCO₃, etc.

Um ein Aktivitätsoptimum der Mikroorganismen zu erreichen, liegt der pH-Wert in den Gelkapseln vorzugsweise zwischen 4 und 9, besonders bevorzugt zwischen 5 und 8 und insbesondere zwischen 6,5 und 8,5. Sofern unter den Anwendungsbedingungen einstellbar, werden die erfindungsgemäßen Starterkulturen vorzugsweise in einem Temperaturbereich von 8°C bis 35°C, besonders bevorzugt in einem Bereich von 15°C bis 30°C und insbesondere zwischen 20°C und 30°C durchgeführt.

In einer weiteren Ausführungsform enthält der Kapselkern und/oder die Kapselwandung Pigmente, z. B. anorganische oder organische Weiß-, Schwarz- oder Buntpigmente und/oder UV-Strahlenfilter. Durch die Verwendung von Pigmenten ist es zum Einen möglich, den Kapseln ein ansprechendes Äußeres zu verleihen, zum Anderen können die Pigmente insbesondere lichtempfindliche Mikroorganismen vor zu starker Licht- bzw. Sonneneinstrahlung schützen. Einen weiteren Schutz vor schädlichen Lichteinwirkungen bieten UV-Strahlenfilter. Die Pigmente und/oder die UV-Strahlenfilter können z. B. gemeinsam mit den Mikroorganismen immobilisiert werden und im Kern vorliegen oder auch in die Kapselwand eingearbeitet werden. Unter diese Ausführungsform fällt auch, daß diese Komponenten sowohl im Kapselinneren in der Matrix, als auch in der Kapselwand vorliegen.

Als Pigmente können beliebige anorganischen oder organische Pigmente verwendet werden, die keine negative Wirkung auf die Aktivität der Mikroorganismen aufweisen. Beispiele für anorganische Pigmente sind Kalk (CaCO₃), Titandioxid, Bleiweiß, Zinkweiß, Lithopone, Antimonweiß, Ruß, Eisenoxidschwarz, Manganschwarz, Cobaltschwarz, Antimonschwarz, Bleichromat, Mennige, Zinkgelb, Zinkgrün, Cadmiumrot, Cobaltblau, Berliner Blau, Ultramarinblau, Manganviolett, Cadmiumgelb, Schweinfurter Grün, Molybdatorange und Molybdatrot, Chromorange und Chromrot, Chromoxidgrün, Strontiumgelb usw., oder in der Natur vorkommende Pigmente wie Ocker, Umbra, Grünerde, Terra di Siena, Graphit usw. Kalk bzw. CaCO₃ hat sich als bevorzugt erwiesen, da es zusätzlich Puffereigenschaften aufweist und auch als CO₂-Quelle für die Mikroorganismen dienen kann.

Die Herstellung der Mikrokapseln kann in an sich bekannter Weise durch Verkapselung von Zellsuspensionen bzw. Lösungen erfolgen.

Sollen Mikrokapseln mit mehreren unterschiedlichen Mikroorganismen hergestellt werden, so werden die Zellsuspensionen/-lösungen, sobald die gewünschte Zellkonzentration eingestellt ist, in den gewünschten Mengenverhältnissen miteinander vermischt und anschließend in an sich bekannter Weise immobilisiert. Geeignete Verfahren zur Immobilisierung der Mikroorganismen sind die aus dem Stand der Technik bekannten Mikroverkapselungsverfahren.

Beispiele für mögliche Herstellungsverfahren sind Phasentrennverfahren, auch Koazervation genannt, mechanisch-physikalische Verfahren, Grenzflächenpolymerisation sowie adsorptive Verfahren.

Koazervation bedeutet, dass ein gelöstes Polymer in eine polymerreiche, noch lösungsmittelhaltige Phase mittels Desolvatation überführt wird. Das Koazervat lagert sich an der Grenzfläche des zu verkapselnden Materials unter Ausbildung einer zusammenhängenden Kapselwand an und wird durch Trocknung oder Polymerisation verfestigt.

Zum Umhüllen fester Kernmaterialien eignen sich auch mechanisch-physikalische Verfahren, worin das Umhüllen in der Wirbelschicht oder durch Sprühtrocknung erfolgt.

In den genannten Grenzflächen-Polymerisationsverfahren erfolgt die Wandbildung durch Polykondensation oder Polyaddition aus monomeren oder oligomeren Ausgangsstoffen an der Grenzfläche einer Wasser/Öl-Emulsion.

Bei den adsorptiven Verfahren werden Schichten aus polyanionischen und polykationischen Polymeren aufgebracht und bilden so die Kapselwand, die üblicherweise aus 2 bis 20 Schichten bestehen kann.

Die eingesetzten Polymere werden vorzugsweise in Form ihrer Lösungen, Suspensionen oder Emulsionen eingesetzt. Für die Mikroverkapselung haben sich wässerige Lösungen, Suspensionen oder Emulsionen mit einer Konzentration von 0,5 bis 10 Gew.-% als geeignet erwiesen. Wie bereits eingangs ausgeführt, ist ein Gesichtspunkt der Anmeldung, vegetative Mikroorganismen in einer solchen Art und Weise zu immobilisieren, dass sie unter ihren Erhaltungsbedinungen über einen langen Zeitraum lagerfähig sind. Um die Lebensfähigkeit der Mikroorganismen, d.h. ihre Aktivität, zu erhalten, ist es daher besonders bevorzugt, wenn die Anzucht, gegebenenfalls die Aufkonzentration und die anschließende Immobilisation unter schonenden Bedingungen, insbesondere unter Erhaltungsbedingungen, durchgeführt werden. Um die Erhaltungsbedingungen einzustellen ist es daher bevorzugt, die Mikroorganismen während des Verarbeitungsprozesses mit Substrat und Sauerstoff zu versorgen. Um als Endprodukt, das üblicherweise dem Endverbraucher selbst zur Verfügung gestellt werden soll, ein funktionsfähiges Produkt zu erhalten, ist es besonders wünschenswert, wenn die genannten Erhaltungsbedingungen lückenlos oder zumindest nahezu lückenlos, während des Herstellungsverfahrens und auch während des Vertriebes bis hin zum Endverbraucher aufrechterhalten werden kann.

Zur Herstellung von Mikrokapseln aus Alginat wird vorzugsweise eine 1 bis 5 %-ige, insbesondere 1,5 bis 2,5 %-ige Alginatsuspension eingesetzt. Diese Alginatsuspension wird mit einer Suspension der Mikroorganismen, die vorzugsweise eine möglichst hohe Konzentration aufweist, vermischt und anschließend in an sich bekannter Weise der Immobilisation unterzogen.

Die hergestellten Immobilisate können ohne weitere Verarbeitungsschritte wie Trocknung, eingesetzt werden. Auch das Trocknen der erhaltenen Immobilisate zum Zwecke der Lagerung ist möglich. Sie können in an sich bekannter Weise dem zu reinigenden und/oder aufzuarbeitenden Wasser eingesetzt werden. Vorzugsweise werden die Kapseln jedoch in ein Behältnis eingebracht, welches in dem zu reinigenden Gewässer fest installiert werden kann. Es ist auch möglich, dass die Immobilisate allein aufgrund ihres spezifischen Gewichts fixiert sind, d.h. von der Strömung nicht weiter getragen werden.

In einer besonders bevorzugten Ausführungsform werden die erhaltenen Immobilisate in einen Filter eingebracht und durch das umgebende Filtermaterial fixiert.

Bei dem Einsatz in einem zu reinigenden Gewässer durchströmt das Wasser die Filtereinheit und kommt mit dem Immobilisate in Kontakt. Durch die vorzugsweise netzartige Struktur des Matrixmaterials dringt das zu reinigende Wasser einschließlich der schädlichen Substanzen in den Innenraum der Immobilisate ein. Durch die Reaktion der immobilisierten Mikroorganismen mit den schädlichen Substanzen erfolgt ein Abbau dieser. Diese für das Wasser schädlichen Substanzen wirken für die Mikroorganismen gleichzeitig als Nährmedium.

Die erfindungsgemäße Verwendung ermöglicht insbesondere die Entfernung von schädlichen anorganische und organischen Wasserinhaltsstoffen, wie Stickstoffverbindungen und Phosphat.

Die verwendeten immobilisierten Mikroorganismen werden vor ihrem erfindungsgemäßen Einsatz vorzugsweise in einem belüfteten und ggf. gekühlten Behältnis gelagert, um einen möglichen Aktivitätsverlust gering zu halten. In einer bevorzugten Ausführungsform ist dieses Behältnis als sog. Spender ausgestaltet, dem die Immobilisate in der gewünschten Menge entnommen werden können.

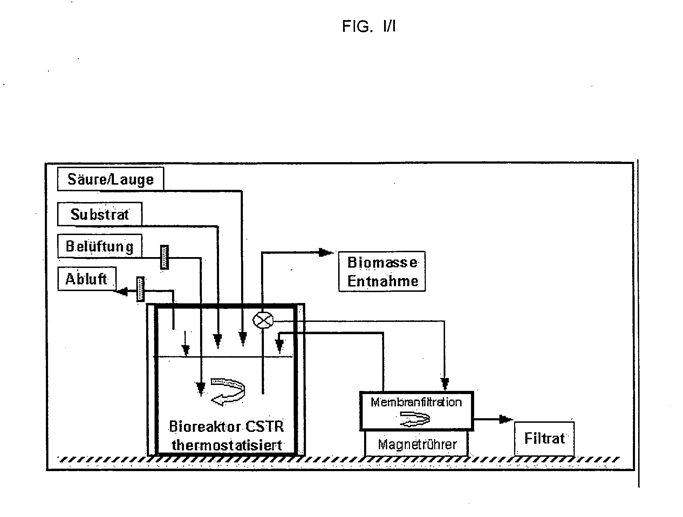

Die beigefügte Figur I zeigt eine schematische Darstellung der Biomasseproduktion für Nitrosomonas sp. als Reinkultur im Bioreaktor mit Biomasserückführung. Die Anzucht erfolgt in Reaktoren mit kontinuierlichen Durchfluß mit Biomasserückführung mittels Membranfiltration zu Endkonzentrationen von 5 x 10⁸ bis 6 x 10⁹ Zellen/ml gezüchtet. Die Anzucht im Fed-Batchsystem ist auch möglich.

## Patentansprüche

1. Verwendung eine mikrobiologische Kultur für die Einleitung von mikrobiologischen Abläufen in der Aquaristik enthaltend lebende chemolithoautotrophe Bakterien die immobilisiert sind, **dadurch gekennzeichnet, dass** die Bakterien in Reinkultur angezüchtet werden und ein Bakteriengemisch aus a) ammoniakoxidierenden und b) nitritoxidierenden eingesetzt wird, wobei das Artenverhältnis der Zellzahlen im Immobilisat von a : b in dem Bereich von 1 : 10.000 bis 1 : 1 liegt.

2. Verwendung nach Anspruch 1, **dadurch gekennzeichnet, dass** die Bakterien in einer Matrix immobilisiert sind, die die Form von Kapseln, Gele und/oder Gelkugeln aufweist.

3. Verwendung nach Anspruch 1 oder 2, **dadurch gekennzeichnet, dass** die Matrix einen Teilchendurchmesser von 1 µm bis etwa 10.000 µm, insbesondere von 100 bis 5.000 µm, aufweist.

4. Verwendung nach einem der Ansprüche 1 bis 3, **dadurch gekennzeichnet, dass** das Matrixmaterial ausgewählt ist aus natürlichen und/oder synthetischen Polymeren.

5. Verwendung nach Anspruch 4, **dadurch gekennzeichnet, dass** die Polymere ausgewählt sind aus polymeren Polysacchariden, wie Agarose oder Cellulose, Proteine, wie Gelatine, Gummi arabicum, Albumin oder Fibrinogen, Ethylcellulose, Methylcellulose, Carboxymethylethylcellulose, Celluloseacetate, Alkalicellulosesulfat, Polyanillin, Polypyrrol, Polyvinylpyrolidon, Polystyrol, Polyvinylchlorid, Polyvinylalkohol, Polyethylen, Polypropylen, Copolymere aus Polystyrol und Maleinsäureanhydrid, Epoxidharze, Polyethylenimine, Copolymere aus Styrol und Methylmethacrylat, Polystyrolsulfonat, Polyacrylate und Polymethacrylate, Polycarbonate, Polyester, Silikone, Methylcellulose, Gemische aus Gelatine und Wasserglas, Gelatine und Polyphosphat, Celluloseacetat und Phthalat, Gelatine und Copolymeren aus Maleinsäureanhydrid und Methylvinylether, Celluloseacetatbutyrat, Chitosan, Polydialkyldimethylammoniumchlorid, Mischungen aus Polyacrylsäuren und Polydiallyldimethylammoniumchlorid sowie beliebige Gemische der voranstehenden

6. Verwendung nach einem der Ansprüche 1 bis 5, **dadurch gekennzeichnet, dass** die Matrix mehrschichtig aufgebaut ist.

7. Verwendung nach Anspruch 6, **dadurch gekennzeichnet, dass** ein Matrixmaterial ausgewählt gelbildenden Polymeren, insbesondere aus Alginaten und/oder Alginatderivaten und ein weiteres Material ausgewählt ist filmbildenden Polymeren, insbesondere aus Alkali-Cellulosesulfat, Polyethyleniminen und/oder Polydialkyldimethylammoniumchloriden.

8. Verwendung nach einem der Ansprüche 1 bis 6, **dadurch gekennzeichnet, dass** dem Matrixmaterial Schicht- und Gerüstsilikate, bevorzugt Zeolithe, zugesetzt werden.

9. Verwendung nach einem der Ansprüche 1 bis 8, **dadurch gekennzeichnet, dass** die Mikroorganismen ausgewählt sind aus chemolithoautotrophen Nitrifikanten, wie den Ammoniakoxidanten und den Nitritoxidanten, wie Bakterien der Gattungen *Nitrosomonas,* Nitrosococcus, *Nitrosospira, Nitrosovibrio und Nitrosospira,* insbesondere die Arten Nitrosomonas halophila, *Nitrosomonas eutropha* und *Nitrosomonas euro*paea, Nitrosomonas oligotropha, Nitrosomonas ureae, Nitrosomonas aestuarii, Nitrosomonas marina, Nitrosomonas sp. 3 Nm 51, Nitrosomonas communis, Nitrosomonas nitrosa, Nitrosomonas sp. 1 Nm 33, Nitrosomonas sp. 2 Nm 41, Nitrosomonas cryotolerans, *Nitrobacter* und *Nitrospira,* insbesondere *Nitrobacter winogradskyi*,
und beliebige Gemische der voranstehenden.

10. Verwendung nach einem der Ansprüche 1 bis 9, **dadurch gekennzeichnet, dass** die Bakterien in Form von Zellsuspensionen immobilisiert werden, wobei die Konzentration der Zellsuspensionen zwischen 5 x 10⁸ bis 6 x 10⁹ Zellen/ml liegt.

11. Verwendung nach einem der Ansprüche 1 bis 10, **dadurch gekennzeichnet, dass** das Bakteriengemisch c) zusätzlich Nitrat- und nitritreduzierenden Bakterien enthält.

12. Verwendung nach Anspruch 11, **dadurch gekennzeichnet, dass** die Nitrat- und Nitrit-reduzierenden Bakterien ausgewählt sind aus heterotrophen Denitrifikanten wie Paracoccus sp.

13. Verwendung nach Anspruch 11 oder 12, **dadurch gekennzeichnet, dass** das Artenverhältnis der Zellzahlen im Immobilisat von a : b in dem 1 : 1.000 bis 1 : 10 liegt.

14. Verwendung nach einem der Ansprüche 11 bis 13, **dadurch gekennzeichnet, dass** das Artenverhältnis von b : c zwischen 1000 : 1 bis 1 : 1 und bevorzugt zwischen 100 : 1 bis 5 : 1 liegt.

15. Verwendung nach einem der Ansprüche 1 bis 14, **dadurch gekennzeichnet, dass** die Matrix ein Puffersystem ausgewählt aus Essigsäure/Acetat, HCO₃⁻/CO₃²⁻, Phosphorsäure/H₂PO₃⁻/HPO₃²⁻, HSO₄⁻/SO₄²⁻, Citronensäure/Citrat, Milchsäure/Lactat, festem CaCO₃ enthält.

## Claims

1. Use of a microbiological culture for triggering microbiological processes in aquaristics, containing living chemolithoautotrophic bacteria which are immobilised, **characterised in that** the bacteria are bred in pure culture and a bacteria mixture consisting of a) ammonia-oxidising bacteria and b) nitrite-oxidising bacteria is used, wherein the type ratio of the cell numbers in the immobilisate of a:b lies in the range from 1:10,000 to 1:1.

2. Use according to Claim 1, **characterised in that** the bacteria are immobilised in a matrix which has the form of capsules, gels and/or gel beads.

3. Use according to Claim 1 or 2, **characterised in that** the matrix has a particle diameter of 1 µm to approximately 10,000 µm, in particular 100 to 5000 µm.

4. Use according to one of Claims 1 to 3, **characterised in that** the matrix material is selected from natural and/or synthetic polymers.

5. Use according to Claim 4, **characterised in that** the polymers are selected from polymeric polysaccharides such as agarose or cellulose, proteins such as gelatin, gum arabic, albumin or fibrinogen, ethylcellulose, methylcellulose, carboxymethylethylcellulose, cellulose acetate, alkali cellulose sulphate, polyaniline, polypyrrole, polyvinylpyrrolidone, polystyrene, polyvinyl chloride, polyvinyl alcohol, polyethylene, polypropylene, copolymers of polystyrene and maleic acid anhydride, epoxy resins, polyethyleneimines, copolymers of styrene and methyl methacrylate, polystyrene sulphonate, polyacrylates and polymethacrylates, polycarbonates, polyesters, silicones, methylcellulose, mixtures of gelatin and water glass, gelatin and polyphosphate, cellulose acetate and phthalate, gelatin and copolymers of maleic acid anhydride and methyl vinyl ether, cellulose acetate butyrate, chitosan, polydialkyldimethylammonium chloride, mixtures of polyacrylic acids and polydiallyldimethylammonium chloride and any mixtures of the above.

6. Use according to one of Claims 1 to 5, **characterised in that** the matrix is of multilayer structure.

7. Use according to Claim 6, **characterised in that** one matrix material is selected from gel-forming polymers, in particular from alginates and/or alginate derivatives, and a further material is selected from film-forming polymers, in particular from alkali cellulose sulphate, polyethyleneimines and/or polydialkyldimethylammonium chlorides.

8. Use according to one of Claims 1 to 6, **characterised in that** phyllosilicates and tectosilicates, preferably zeolites, are added to the matrix material.

9. Use according to one of Claims 1 to 8, **characterised in that** the microorganisms are selected from chemolithoautotrophic nitrifying bacteria, such as ammonia-oxidising bacteria and nitrite-oxidising bacteria, such as bacteria of the genus Nitrosomonas, Nitrosococcus, Nitrosospira, Nitrosovibrio and Nitrosospira, in particular the species Nitrosomonas halophila, Nitrosomonas eutropha and Nitrosomonas europaea, Nitrosomonas oligotropha, Nitrosomonas ureae, Nitrosomonas aestuarii, Nitrosomonas marina, Nitrosomonas sp. 3 Nm 51, Nitrosomonas communis, Nitrosomonas nitrosa, Nitrosomonas sp. 1 Nm 33, Nitrosomonas sp. 2 Nm 41, Nitrosomonas cryotolerans, Nitrobacter and Nitrospira, in particular Nitrobacter winogradskyi, and any mixtures of the above.

10. Use according to one of Claims 1 to 9, **characterised in that** the bacteria are immobilised in the form of cell suspensions, wherein the concentration of the cell suspensions is between 5 × 10⁸ and 6 × 10⁹ cells/ml.

11. Use according to one of Claims 1 to 10, **characterised in that** the bacteria mixture additionally contains c) nitrate- and nitrite-reducing bacteria.

12. Use according to Claim 11, **characterised in that** the nitrate- and nitrite-reducing bacteria are selected from heterotrophic denitrifying bacteria such as Paracoccus sp.

13. Use according to Claim 11 or 12, **characterised in that** the type ratio of the cell numbers in the immobilisate of a:b is in the range from 1:1000 to 1:10.

14. Use according to one of Claims 11 to 13, **characterised in that** the type ratio of b:c is between 1000:1 and 1:1 and preferably between 100:1 and 5:1.

15. Use according to one of Claims 1 to 14, **characterised in that** the matrix contains a buffer system selected from acetic acid/acetate, HCO₃⁻/CO₃²⁻, phosphoric acid/H₂PO₃⁻/HPO₃²⁻, HSO₄⁻/SO₄²⁻, citric acid/citrate, lactic acid/lactate, solid CaCO₃.

## Revendications

1. Utilisation d'une culture microbiologique pour l'introduction de processus microbiologiques dans des aquariums, contenant des bactéries chimiolitho-autotrophes vivantes qui sont immobilisées, **caractérisée en ce que** les bactéries sont cultivées en culture pure, et **en ce que** l'on utilise un mélange de bactéries constitué de bactéries a) oxydant l'ammoniaque et b) oxydant les nitrites, le rapport des espèces a/b en nombre de cellules dans l'immobilisat se situant dans la plage de 1/10 000 à 1/1.

2. Utilisation selon la revendication 1, **caractérisée en ce que** les bactéries sont immobilisées dans une matrice, qui prend la forme de capsules, de gels et/ou de billes de gel.

3. Utilisation selon la revendication 1 ou 2, **caractérisée en ce que** la matrice présente un diamètre de particules de 1 µm à environ 10 000 µm, en particulier de 100 à 5000 µm.

4. Utilisation selon l'une quelconque des revendications 1 à 3, **caractérisée en ce que** la matière de la matrice est choisie parmi les polymères naturels et/ou synthétiques.

5. Utilisation selon la revendication 4, **caractérisée en ce que** les polymères sont choisis parmi les polysaccharides polymères, comme l'agarose ou la cellulose, les protéines comme la gélatine, la gomme arabique, l'albumine ou le fibrinogène, l'éthylcellulose, la méthylcellulose, la carboxyméthyléthylcellulose, les acétates de cellulose, le sulfate d'alcalicellulose, la polyaniline, le polypyrrole, la polyvinylpyrrolidone, le polystyrène, le chlorure de polyvinyle, l'alcool polyvinylique, le polyéthylène, le polypropylène, les copolymères de polystyrène et d'anhydride d'acide maléique, les résines époxyde, les polyéthylènimines, les copolymères de styrène et de méthacrylate de méthyle, le sulfonate de polystyrène, les polyacrylates et polyméthacrylates, les polycarbonates, les polyesters, les silicones, la méthylcellulose, les mélanges de gélatine et de verre soluble, la gélatine et le polyphosphate, l'acétate et le phtalate de cellulose, la gélatine et les copolymères d'anhydride d'acide maléique et d'éther méthylvinylique, le butyrate d'acétate de cellulose, le chitosane, le chlorure de polydialkyldiméthyl-ammonium, les mélanges d'acides polyacryliques et de chlorure de polydiallyldiméthylammonium, ainsi que des mélanges quelconques des polymères précédents.

6. Utilisation selon l'une des revendications 1 à 5, **caractérisée en ce que** la matrice a une structure multicouche.

7. Utilisation selon la revendication 6, **caractérisée en ce qu'**une matière de matrice est choisie parmi les polymères gélifiants, en particulier les alginates et/ou les dérivés d'alginates, et une autre matière est choisie parmi les polymères filmogènes, en particulier parmi le sulfate d'alcalicellulose, les polyéthylènimines et/ou les chlorures de polydialkyldiméthylammonium.

8. Utilisation selon l'une des revendications 1 à 6, **caractérisée en ce que** la matière de matrice est additionnée de silicates lamellaires et tectosilicates, de préférence des zéolithes.

9. Utilisation selon l'une des revendications 1 à 8, **caractérisée en ce que** les micro-organismes sont choisis parmi les nitrifiants chimiolitho-autotrophes comme les oxydants d'ammoniaque et les oxydants de nitrite, comme les bactéries des genres *Nitrosomonas, Nitrosococcus, Nitrosospira, Nitrosovibrio* et *Nitrosospira,* en particulier les espèces *Nitrosomonas halophila, Nitrosomonas eutropha* et *Nitrosomonas europaea, Nitrosomonas oligotropha, Nitrosomonas ureae, Nitrosomonas aestuarii, Nitrosomonas marina, Nitrosomonas sp 3 Nm 51, Nitrosomonas communis, Nitrosomonas nitrosa, Nitrosomonas sp 1 Nm 33, Nitrosomonas sp 2 Nm 41, Nitrosomonas cryotolerans, Nitrobacter* et *Nitrospira,* en particulier *Nitrobacter winogradskyi,* et des mélanges quelconques des micro-organismes précédents.

10. Utilisation selon l'une des revendications 1 à 9, **caractérisée en ce que** les bactéries sont immobilisées sous forme de suspensions cellulaires, la concentration des suspensions cellulaires se situant entre 5 x 10⁸ à 6 x 10⁹ cellules/ml.

11. Utilisation selon l'une des revendications 1 à 10, **caractérisée en ce que** le mélange de bactéries contient en plus c) des bactéries réduisant les nitrates et les nitrites.

12. Utilisation selon la revendication 11, **caractérisée en ce que** les bactéries réduisant les nitrates et les nitrites sont choisies parmi les dénitrifiants hétérotrophes comme *Paracoccus sp.*

13. Utilisation selon la revendication 11 ou 12, **caractérisée en ce que** le rapport des espèces a/b en nombre de cellules dans l'immobilisat va de 1/1000 à 1/10.

14. Utilisation selon l'une des revendications 11 à 13, **caractérisée en ce que** le rapport des espèces b/c se situe entre 1000/1 et 1/1 et de préférence entre 100/1 et 5/1.

15. Utilisation selon l'une des revendications 1 à 14, **caractérisée en ce que** la matrice contient un système tampon choisi parmi acide acétique/acétate, HCO₃⁻/CO₃²⁻, acide phosphorique/H₂PO₃⁻/HPO₃²⁻, HSO₄⁻/SO₄²⁻, acide citrique/citrate, acide lactique/lactate, CaCO₃ solide.
